# EUROPEAN PATENT APPLICATION

(11) **EP 2 883 505 A1**
(43) Date of publication of application: **17.06.2015**
(21) Application number: 13879426.8
(22) Date of filing: 12.08.2013
(51) Int. Cl.: A61B 17/06

(54) **NEEDLE EQUIPPED SUTURE AND MANUFACTURING METHOD THEREFOR**

(30) Priority: 13.08.2012 JP 2012179308
(71) Applicant: MANI, INC., Utsunomiya-shi, Tochigi 321-3231 (JP)
(72) Inventor: AKATSUKA, Masao, Utsunomiya-shi Tochigi 321-3231 (JP)
(74) Representative: Isarpatent
(86) International application number: PCT/JP2013/071787
(87) International publication number: WO 2014/027634

(57) **Abstract**

There is provided a manufacturing method for a suture thread with suture needle that has little dispersion in pull-out load and is user-friendly.

A blind hole (14) is opened along the length of an eyeless suture needle (10) from a base end surface (13) of the eyeless suture needle (10), a suture thread (18) is inserted in the blind hole (14), and then caulked and formed. A caulking force according to size of diameter of the blind hole (14) is added, cross-sectional form of a caulking part is nearly circular, and the axial length of the caulking part is less than the diameter of the eyeless suture needle (10). According to such a structure, the suture thread (18) may be pulled out from the eyeless suture needle due to a pull-out load within a predetermined force range, and dispersion in pull-out force may be reduced.

## Description

### [Technical Field]

The present invention relates to a suture thread with suture needle where the suture thread is attached to an eyeless suture needle. It particularly relates to a suture thread with suture needle where connecting force between the suture thread and the suture needle is within a predefined range, and the suture needle can be pulled out when a tensile force of a certain level or more is applied, and a manufacturing method for the same.

### [Background Art]

There are many kinds of medical suture needles according to intended purpose. Representative examples thereof are cutting needles and taper point needles. A cutting needle is generally used for suturing hard tissue such as skin, muscle, etc., has a sharp, pointy front end, a polygonal pyramid-shaped tapered part, and a body part that is formed on the base end side thereof and has a predetermined cross-sectional form; and predetermined ridges of the tapered part are cutting blades so as to cut open tissue. The cross-sectional form of the body part may be polygonal such as triangular, square, as well as circular or oval.

The taper point needle has a front end, a conical tapered part, and a body part that is formed on the base end side and has a predetermined cross-sectional form. The cross-sectional forms of the conical tapered part and the body part may be circular, oval having two nearly parallel sides, a form having four parallel sides, the form of an hourglass, etc. However, the ridges are not sharpened and used as cutting blades as with the cutting needle.

The taper point needle bores a hole in the tissue by its own needlepoint end and the hole is then widened by its own tapered part; however, since it does not have a cutting blade as the cutting needle, the tissue can not to be cut recklessly. Therefore, biomedical tissue at the open hole part adhering to the surface of the thread and the bodily fluid etc. leaking from the suturing part may be prevented. Due to such properties, the taper point needle is used in suturing mainly blood vessels and soft tissue.

The medical suture needle is generally manufactured in the following manner. A wire rod having a circular cross section with a predetermined thickness is first cut to a predetermined length. An end of this material is then formed into a catching part for catching a suture thread. The catching part may be a blind hole opened along the axis or a split eye, for example. In the case of the cutting needle, once the material is press worked into a predetermined cross-sectional form and formed into a body part, cutting blades are formed through a grinding process and a tapered part and a sharp front end are formed. In the case of the taper point needle, a sharp front end portion is ground using a grindstone or the like and a tapered part without a cutting blade is formed. Once coarse buffing or grinding using small whetstones or the like is performed, grinding marks are removed by fine buffing, electrolytic buffing, etc. so as to finish the mirror surface and bent into a predetermined form, and heat treatment and surface treatment are performed as necessary, thereby completing the suture needle.

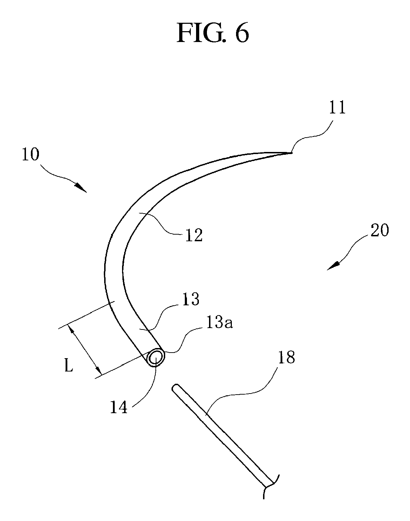

FIG. 6 illustrates a taper point needle as a conventional example of a medical eyeless suture needle. An eyeless suture needle 10, as mentioned above, includes a sharp, pointy front end 11, a tapered part 12 extending conically from the front end 11, and a base end 13 having a predetermined cross-sectional form and formed on the base end side thereof. The cross-sectional forms of the conical tapered part 12 and the base end 13 may be circular, oval having two nearly parallel sides, a form having four parallel sides, a form of an hourglass, etc.

The eyeless suture needle 10 has a blind hole 14 as a catching part for catching a suture thread 18 along the length at a base end surface 13a or end surface of the base end 13. The blind hole 14 may be opened through laser processing of opening a hole by irradiating a laser beam, electronic beam processing of converting kinetic energy to thermal energy by making a needle material collide into a physical matter accelerated to a high speed in a vacuum and fusing it instantly so as to open a hole, electric discharge machining of discharging electricity between an electrode having the same diameter as the hole and a workpiece and processing the workpiece, or drilling, which opens a hole in an end surface of the suture needle using a slim drill. The blind hole 14 generally has a depth of twice or more than the diameter of the eyeless suture needle 10.

As the suture thread 18, many different types of threads such as those differing in thickness or material (nylon, silk, etc.), or having a monofilament or multifilament structure, etc. are provided. The appropriate type of thread is selected for biomedical tissue to be sutured and a suturing location and then used. Since when the suture thread 18 is a twisted thread or knitting yarn, the end is frayed when cut, either the thread is pulled before cutting, applied with a resin and then cut after it has hardened, or, in the case of a material that hardens through heating, an end thereof is heated to harden and then cut.

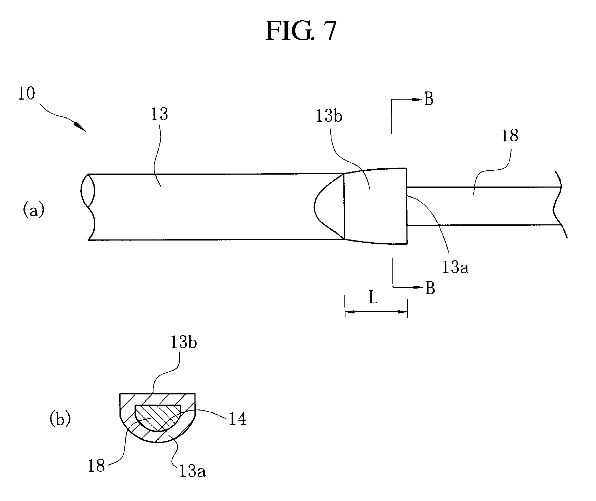

When the blind hole 14 is opened, the front end of the suture thread 18 is inserted in the blind hole 14, and the blind hole 14 is crushed by a pressing machine or the like and caulked, thereby fixing the suture thread 18 to the base end 13 of the eyeless suture needle 10, and resulting in a suture thread with suture needle 20. FIG. 7 shows magnified views of the base end 13 of the caulked suture thread with suture needle 20; where 7(a) is a front view and 7(b) is a cross-sectional view cut along the line B-B of 7(a). The suture thread with suture needle 20 has an advantage that the trouble of passing thread through the eye of, for example, an eyed needle is unnecessary since a required length of the suture thread 18 is fixed to the suture needle 10 from the beginning. The caulking length of the blind hole 14 is approximately two-thirds the depth thereof. As described above, since the depth of the blind hole 14 is generally double or more of the diameter of the eyeless suture needle 10, the caulking length is longer (length of approximately four-thirds or more of the diameter of the eyeless suture needle 10) than the diameter of the eyeless suture needle 10.

In suturing using the suture thread with suture needle 20, the front end 11 of the suture needle 10 is first pierced through one side of a cut line or the like of a biomedical tissue to be sutured, and the front end 11 is then pushed in so as to pass the cut line and thrust out from the other side until it reaches the tapered part 12. The thrust out tapered part 12 is grabbed using a needle holder so as to extract the suture needle 10 and pull out the suture thread 18 connected to the suture needle 10. When an appropriately long enough part of the suture thread 18 is pulled out, it is tied to the remaining suture thread 18 on the other side of the cut line and the excess suture thread 18 is cut using scissors.

When wanting to shorten suturing time such as in the case of suturing many times, once a surgeon pushes the suture needle through and extracts it, he/she pulls the suture thread out until it is an appropriate length, applies tensile force between the suture thread and the suture needle and thus pulls the suture needle out from the suture thread and removes only the suture needle. This allows shortening of a required amount of time for a suture operation.

A suture thread with suture needle that allows the suture thread to be pulled out from the suture needle when a certain degree of tensile force or more is applied for such a purpose is well known (e.g., Patent Document 1). However, if it can be pulled out even with a small tensile force, there is a chance that the suture thread comes out during the suture operation, and thus making sure that it does not come out until a certain degree of tensile force or more is applied is necessary.

Patent Document 1 describes a suture thread with a needle having a pull-out load larger than 3 ounces and smaller than 26 ounces. In order to achieve such an aim, the diameters of needle blind holes and suture threads should be uniform. It describes that one skilled in the art can keep the diameters of needle blind holes within a range of ±0.0005 inches (12.7 µm) when opening the holes using a drill. Meanwhile, regarding uniform diameters of suture threads, it describes that by keeping braided suture threads tense and immersing the portion that will be inserted into the needle blind hole in an adhesive or a binding resin and hardening it, fraying may be prevented and a constant thickness may be maintained.

### [Prior Art Documents]

### [Patent Documents]

Patent Document 1: JP S50-73480A

### [Disclosure of Invention]

### [Problem to be Solved by the Invention]

However, the suture thread with suture needle of Patent Document 1 has a pull-out load that is larger than 3 ounces (85.05g) and smaller than 26 ounces (737.1g). That is, difference between the minimum and the maximum pull-out load is 652.05g. There is a problem that it is not easy to handle due to a considerable degree of dispersion.

The present invention is devised to resolve this problem, and aims to provide a user-friendly suture thread with suture needle with little dispersion in pull-out load.

### [Means of Solving the Problem]

A suture thread with suture needle of the present invention for achieving the above aim is characterized by having a blind hole opened along the length of an eyeless suture needle from a base end surface of the eyeless suture needle, and a suture thread inserted in the blind hole and caulked and formed so that the suture thread can be pulled out from the eyeless suture needle according to a pull-out load within a predetermined force range. Cross-sectional form of a caulking part is nearly circular, the axial length of the caulking part that is provided from the base end surface of the eyeless suture needle is less than the diameter of the eyeless suture needle, and an end of the suture thread is positioned further in the blind hole than the caulking part. Here, 'nearly circular cross-section' refers to proportion of two diameters within the range of 0.95 to 1.05 with rounded corners or without corners when the diameter of the caulking part is measured in two mutually orthogonal directions. Moreover, the blind hole is preferably opened using a laser, multiple eyeless suture needles are classified into multiple groups, each increasing in blind hole diameter length by 5 µm, and the caulking amount of each group is kept constant.

### [Result of the Invention]

According to the present invention, beneficial effects such as allowing reduction in dispersion of pull-out load, stable extraction, and shortening time required for suturing are brought about. Moreover, since the cross-sectional form of the caulking part is nearly circular, there are no corners, thereby reducing damage to the biomedical tissue when suturing. Furthermore, the caulking part is provided from the base end, the axial length of the caulking part is smaller than diameter of the suture needle, and an end of the suture thread is positioned further in the blind hole than the caulking part, and thereby dispersion is reduced and damage to the biochemical tissue is also reduced. Moreover, the blind hole is opened using a laser, multiple eyeless suture needles are classified into multiple groups, each increasing in blind hole diameter length by 5 µm, the caulking amount of each group is kept constant, and dispersion is reduced

### [Brief Description of Drawings]

FIG. 1 illustrates the blind hole diameter of a suture needle of the present invention; where 1(a) shows a case of a smaller hole diameter than a standard, 1(b) shows a case of a standard hole diameter, and 1(c) shows a case of a larger hole diameter than the standard;
FIG. 2 is a drawing illustrating a die used for caulking;
FIGS. 3(a) to 3(d) are diagrams describing a caulking operation;
FIG. 4 is a diagram illustrating an external view of a base end and a caulking part of a caulked suture needle;
FIG. 5 is a diagram illustrating a modified example of the caulking part according to the present invention;
FIG. 6 is an oblique view of a taper point needle as a conventional example of a medical eyeless suture needle; and
FIG. 7 shows magnified views of the base end of the caulked suture thread with suture needle; where 7(a) is a front view and 7(b) is a cross-sectional view cut along the line B-B of 7(a).

### [Best Mode for Carrying Out the Invention]

An embodiment according to the present invention is described below with reference to accompanying drawings.

The present invention has characteristics in that a suture thread can be extracted from an eyeless suture needle by a pull-out load within a predetermined range, resulting from inserting a suture thread in a blind hole 14 opened in the base end surface 13a of an eyeless suture needle, and caulking a caulking part so that the axial length of the caulking part that has a nearly circular cross-sectional form and is provided from a base end of the eyeless suture needle becomes less than the diameter of the eyeless suture needle, and that an end of the suture thread becomes positioned further in the blind hole 14 than the caulking part.

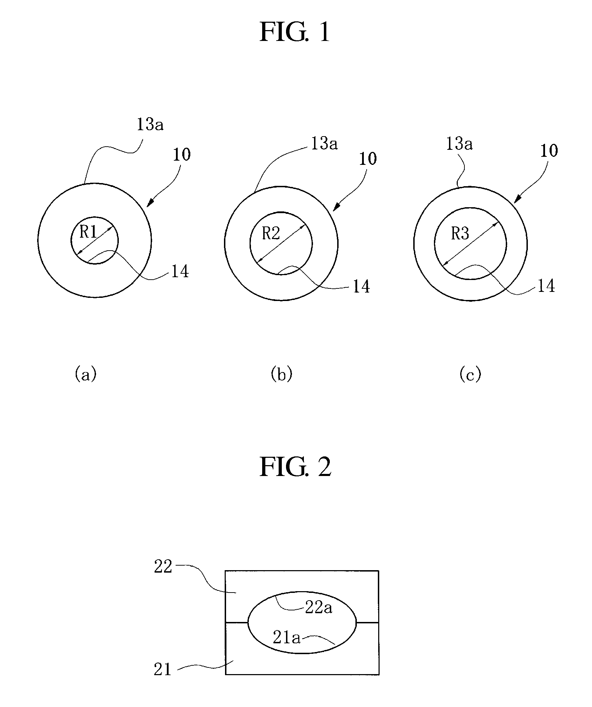

FIG. 1 illustrates a state of the blind hole 14 opened along the needle length in the base end surface 13a of the eyeless suture needle 10 in a manufacturing process of the suture needle of the present invention. The opening method of the blind hole 14 is not particularly limited, but laser processing is employed in this embodiment. The diameter of the suture needle is 0.98 mmϕ and standard diameter of the blind hole 14 is 0.47 mmϕ (470 µm). Moreover, the suture thread 18 has had an end for binding to the suture needle 10, heated and hardened in order to prevent scuffing.

In the embodiment of the present invention, the blind hole 14 is opened through laser processing. While accuracy of hole diameter increases through drilling, a hole may be opened in a shorter time with the laser processing and has good output efficiency, and is therefore employed. The present invention has a characteristic in that dispersion in pull-out load can be reduced even when using the laser processing to open a hole.

FIG. 1(a) shows a group where diameter R1 of the blind hole 14 is smaller than a standard and the blind hole 14 accommodates a 465 µmϕ gauge but does not accommodate a 470 µmϕ gauge. FIG. 1(b) shows a group case where diameter R2 of the blind hole 14 is the standard and the blind hole 14 accommodates a 470 µmϕ gauge but does not accommodate a 475 µmϕ gauge. FIG. 1(c) shows a group where diameter R3 of the blind hole 14 is larger than the standard and the blind hole 14 accommodates a 475 µmϕ gauge but does not accommodate a 480 µmϕ gauge. Such grouping is carried out so that dispersion in diameter of the blind hole 14 is 5 µm or less.

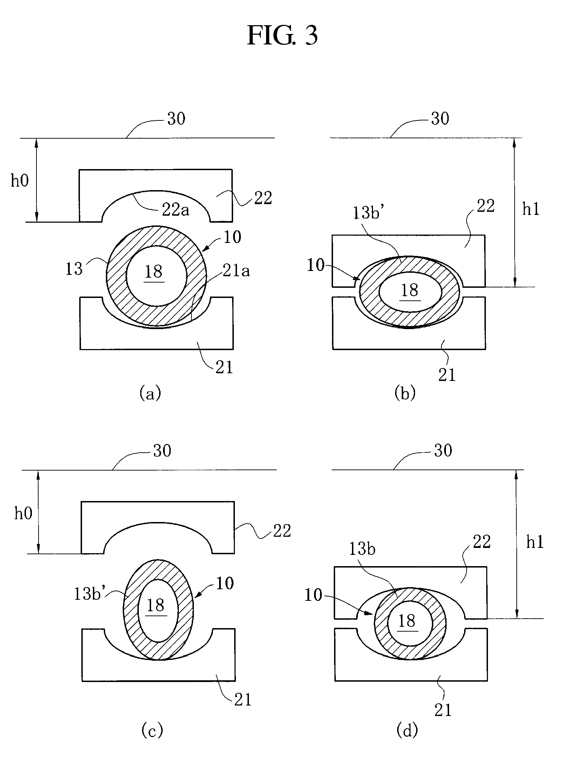

FIG. 2 is a drawing illustrating a die used for caulking. The die is constituted by a lower die 21 and an upper die 22. The lower die 21 and the upper die 22 have a half elliptic concave portion 21a and 22a, respectively, and stacking the lower die 21 and the upper die 22 forms an elliptic-shaped hole using the two concave portions 21a and 22a, as illustrated in FIG. 3.

FIG. 3 is a drawing for describing a caulking operation. As shown in FIG. 3(a), first, the base end surface 13a of the base end 13 of the suture needle 10 is inserted between the upper and lower dies 21 and 22 so that the axial length of a portion that becomes the caulking part is less than the diameter of the eyeless suture needle 10. FIG. 3(a) shows a state where the suture thread 18 is inserted in the blind hole 14. At this time, the end of the suture thread 18 is positioned further inward than the portion that becomes the caulking part. FIG. 3(b) shows a state where a first caulking operation is finished and the base end 13 is thus caulked into an elliptic form. At this time, as a general rule, the upper die 22 and the lower die 21 are in an unattached state with space therebetween. Next, as shown in FIG. 3(c), the upper and lower dies 21 and 22 are pulled apart, and the base end 13 caulked into an elliptic form in the first caulking operation is rotated 90 degrees and inserted therein. Then, as shown in FIG. 3(d), a second caulking operation is performed and thus the base end 13 is a caulking part 13b having a circular cross-sectional form. The base end 13 of the suture needle and caulking parts 13b' and 13b are hatched in these drawings for facilitation of visualization but displaying cross sections.

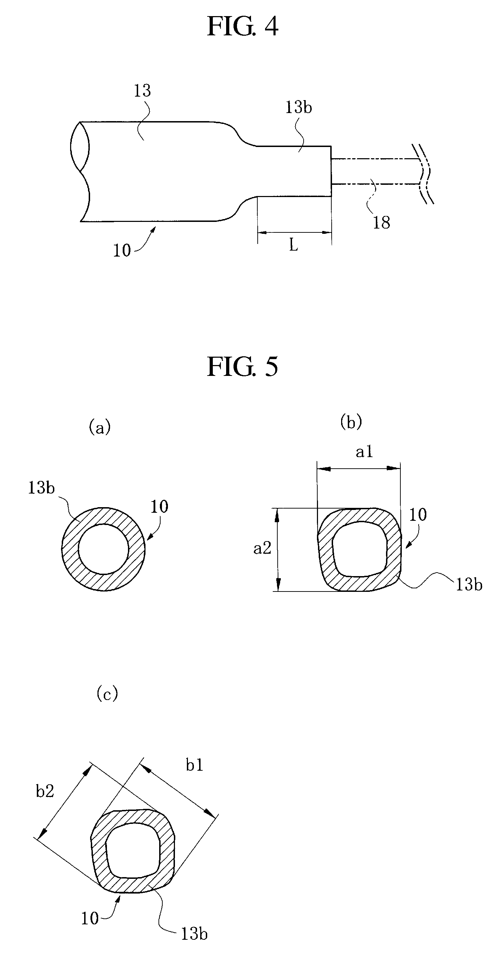

FIG. 4 is a diagram illustrating an external view of the base end 13 and the caulking part 13b of the caulked suture needle 10. With the present invention, as shown in FIG. 3(d), the caulking operation is performed twice in a perpendicular direction to the die having an elliptical concave portion in order to make the caulking part 13b have a circular cross-sectional form. Note that the caulking part 13b is formed from an end of the base end 13 and axial length L is 0.5 mm in all cases. In doing so, the end of the suture thread 18 is positioned further in the blind hole 14 from the caulking part 13b and positioning of the suture thread 18 along the entire length L of the caulking part 13b is assured. Formation of the caulking part 13b from the end of the base end 13 allows further reduction in dispersion regardless of the hole opening method such as drilling, laser processing, etc. This is because there is less dispersion in diameter when opening a hole on the end side of the base end. For example, with laser processing, hole form often has a spindle shape where the center of the hole is swollen, and therefore the end side has less dispersion in diameter. Furthermore, since there are fewer steps in forming a hole from the end of the base end 13, damage to the biomedical tissue is reduced. According to an experiment by the inventor, little dispersion was obtained in the case of the axial length L of the caulking part 13b being less than the diameter of the suture needle 10, particularly in the case of it being approximately 40 to 90% of the diameter of the suture needle 10.

FIG. 5 illustrates a modified example of the caulking part 13b according to the present invention. FIG. 5(a) shows the case of a circular cross-section and FIG. 5(b) shows the case of a cross-sectional form of a square with rounded corners. While the cross-sectional form of the caulking part 13b nearly becomes a circular form when caulking illustrated in FIG. 3 is used, it does not always become a circular form. When the diameter of the caulking part 13b in the case of the form in FIG. 5(b) is measured in two mutually orthogonal directions and values of a1 and a2 are found, the caulking part according to the present invention is included within the range of a1 / a2 = 0.95 to 1.05, where with the present invention, within the range of a1 / a2 = 0.95 to 1.05 with rounded corners or without corners is defined as 'nearly circular form'.

The diameter of the cross-sectional form shown in FIG. 5(b) may also be measured by the method illustrated in FIG. 5(c). In this case, the diameter is also included within the range of b1 / b2 = 0.95 to 1.05 when measured in two mutually orthogonal directions.

When inserting the suture thread 18 in the blind hole 14 and caulking it, there is a method of controlling using a load by a pressing machine, and a method of controlling using caulking amount. With the present invention, controlling using caulking amount is employed.

With the pressing machine illustrated in FIG. 3, a configuration where the lower die 21 is fixed and the upper die 22 rises is employed. A reference position 30 is unique to the pressing machine, and h0 shown in FIG. 3(a) and FIG. 3(c) denotes distance from an initial position and a reference position of the upper die 22. At the same time, h1 shown in FIG. 3(b) and FIG. 3(d) denotes distance from the reference position 30 to a stop position of a bottom surface of the upper die 22 when the first and second caulking operations are completed. When the distance h1 is larger, the diameter of the caulking part 13b becomes smaller, and when h1 is smaller, it becomes larger. That is, since the diameter of the caulking part 13b is determined according to the distance h1, the distance h1 is defined as caulking amount in the present application.

If the caulking amount h1 is increased, the blind hole 14 is crushed more and the pull-out load is increased, and if the caulking amount h1 is decreased, the blind hole is crushed less and the pull-out load is decreased. Since the diameter of the suture needle 10 in the embodiment of the present invention is 0.98 mm, a pull-out load of around 336g is preferable for the suture needle having this diameter. This value is determined according to an empirical value etc.

FIG. 1(a) shows a group where the diameter of the blind hole 14 is smaller than the standard and the blind hole 14 accommodates a 465 µmϕ gauge but does not accommodate a 470 µmϕ gauge. The hardened end of the suture thread 18 is inserted into the blind hole 14 of the suture needle 10 having the blind hole 14 of this size so that the end is positioned further inward than the portion to become the caulking part, the suture needle 10 is positioned between the dies 21 and 22 such that the axial length of the portion to become the caulking part is less than the diameter of the suture needle 10, and as shown in FIG. 3, a caulking operation is performed twice thereby obtaining a nearly circular cross-sectional form. Upon varying the values of the caulking amount h1 diversely and finding an average value and a standard deviation of ten pull-out loads,
when h1 = 34.061 mm,
maximum pull-out load = 480g
average pull-out load = 367g
minimum pull-out load = 205g
standard deviation = 100g
From the above, the group for this hole diameter is determined as having a caulking amount h1 of 34.061 mm.

As can be understood from the above data, the caulking amount according to the present invention needs to be controlled in units of µm. This aim cannot be achieved with a typical pressing machine, and a pressing machine that can accurately set distance to be pressed in units of µm is required. In order to control the caulking amount in units of µm with the present invention, grouping and then caulking are carried out so that dispersion in diameter of the blind hole 14 can be within 5 µm, and at this time, dispersion in diameter of the caulking part 13b is also 5 µm or less.

FIG. 1(b) shows a group where the diameter of the blind hole 14 is the standard and the blind hole 14 accommodates a 470 µmϕ gauge but does not accommodate a 475 µmϕ gauge. The hardened end of the suture thread 18 is inserted into the blind hole 14 of the suture needle 10 having the blind hole 14 of this size so that the end is positioned further inward than the portion to become the caulking part, the suture needle 10 is positioned between the dies 21 and 22 such that the axial length of the portion to become the caulking part is less than the diameter of the suture needle 10, and as shown in FIG. 3, a caulking operation is performed twice, thereby obtaining a nearly circular cross-sectional form.

Upon varying the values of h1 diversely and finding an average value and a standard deviation of ten pull-out loads,
when h1 = 34.038 mm,
maximum pull-out load = 586g
average pull-out load = 367g
minimum pull-out load = 113g
standard deviation = 91g
From the above, the group for this hole diameter is determined as having a caulking amount h1 of 34.038 mm.

FIG. 1(c) shows a size group where the diameter of the blind hole 14 is larger than the standard and the blind hole 14 accommodates a 475 µmϕ gauge but does not accommodate a 480 µmϕ gauge. The hardened end of the suture thread 18 is inserted into the blind hole 14 of the suture needle 10 having the blind hole 14 of this size so that the end is positioned further inward than the portion to become the caulking part, the suture needle 10 is positioned between the dies 21 and 22 such that the axial length of the portion to become the caulking part is less than the diameter of the suture needle 10, and as shown in FIG. 3, a caulking operation is performed twice, thereby obtaining a nearly circular cross-sectional form.

Upon varying the values of h1 diversely and finding an average value and a standard deviation of ten pull-out loads,
when h1 = 34.120 mm,
maximum pull-out load = 486g
average pull-out load = 317g
minimum pull-out load = 98g
standard deviation = 79g
From the above, the group for this hole diameter is determined as having a caulking amount h1 of 34.120 mm.

In the above embodiment, while caulking is performed such that the cross-sectional form becomes a nearly circular form, making this circular cross section, caulking from the end of the base end, making the length of the caulking part 13b be less than the diameter of the suture needle 10, and classifying multiple eyeless suture needles into groups according to diameter length allow reduction of dispersion in pull-out load. Furthermore, since the cross-sectional form of the caulking part 13b is nearly circular, no formation of corners is made on the caulking part 13b, and thus damaging an affected area by the caulking part 13b when suturing the affected area may be prevented.

Moreover, while it is desirable to measure the size of the diameter of the blind hole 14 for each suture needle and set the caulking amount according to size of each diameter, operating efficiency is decreased. Therefore, the method of dividing diameters of the blind hole 14 into groups and keeping the caulking amount for each group constant is employed. Such grouping by 5 µm is merely an example. The smaller the difference between blind hole groups, the less the dispersion may be.

In the above embodiment, the caulking amount is determined by the distance h1 from the reference position to the stop position of the upper die 22; however, another method, such as determining by directly measuring the diameter of the caulking part may be employed.

### [Description of Reference Numerals]

10: Eyeless suture needle
11: Needlepoint
12: Tapered part
13: Base end
13a: Base end surface
14: Blind hole
18: Suture thread
20: Suture thread with suture needle
21: Lower die
22: Upper die
30: Reference position
h1: Caulking amount

## Claims

1. A suture thread with suture needle that has: a blind hole opened along the length of an eyeless suture needle from a base end surface of the eyeless suture needle; and a suture thread inserted in the blind hole and caulked and formed so that the suture thread can be pulled out from the eyeless suture needle according to a pull-out load within a predetermined force range; wherein cross-sectional form of a caulking part is nearly circular, the axial length of the caulking part that is provided from the base end surface of the eyeless suture needle is less than the diameter of the eyeless suture needle, and an end of the suture thread is positioned further in the blind hole than the caulking part.

2. The suture thread with suture needle of Claim 1, wherein the blind hole is opened using a laser, multiple eyeless suture needles are classified into multiple groups, each increasing in blind hole diameter length by 5 µm, and the caulking amount of each group is kept constant.

3. A manufacturing method for a suture thread with suture needle that has: a blind hole opened along the length of an eyeless suture needle from a base end surface of the eyeless suture needle; and a suture thread inserted in the blind hole and caulked and formed so that the suture thread can be pulled out from the eyeless suture needle according to a pull-out load within a predetermined force range, said manufacturing method comprising the steps of:
opening the blind hole using a laser;
classifying multiple eyeless suture needles into multiple groups, each increasing in blind hole diameter length by 5 µm; and
inserting an end of the suture thread in the blind hole so as for the end to position further inward than a caulking part; and
caulking such that respective caulking amounts of each group are kept constant, cross-sectional form of the caulking part is nearly circular, and the axial length of the caulking part that is provided from the base end surface of the eyeless suture needle is less than the diameter of the eyeless suture needle.
